# EUROPEAN PATENT APPLICATION

(11) **EP 4 763 237 A1**
(43) Date of publication of application: **24.06.2026**
(21) Application number: 25226114.4
(22) Date of filing: 22.12.2025
(51) Int. Cl.: A61L 26/00

(54) **HYDROGEL DRESSING CONTAINING BIOACTIVE SUBSTANCES AND METHOD FOR ITS MANUFACTURE**

(30) Priority: 22.12.2024 PL 45066724
(71) Applicant: Uniwersytet Rolniczy im. Hugona Kollataja w Krakowie, 31-120 Kraków (PL); Akademia Gorniczo-Hutnicza im. Stanislawa Staszica w Krakowie, 30-059 Krakow (PL); Uniwersytet Jagiellonski, 31-007 Krakow (PL)
(72) Inventor: Molik, Edyta, 30-126 Krakow (PL); Szatkowski, Piotr, 31-559 Krakow (PL); Ptak, Anna, 32-406 Czechowka (PL)
(74) Representative: Czarnik, Maciej

(57) **Abstract**

A hydrogel dressing comprising at least one layer of a hydrogel matrix with at least one bioactive substance, characterized in that the said hydrogel matrix contains from 4 to 8 wt.% of an aqueous solution of polyvinyl alcohol (PVA) and 4 wt.% of an aqueous solution of sodium tetraborate (borax), and the bioactive substance is sheep milk in an amount of from 5 to 20 wt.% and/or camel milk in an amount of from 2.5 to 15 wt.%.The invention further relates to a method of producing the hydrogel dressing.

## Description

The subject matter of the invention relates to a hydrogel dressing comprising bioactive substances and to a method for producing the same. The invention further relates to the use of a hydrogel dressing comprising bioactive substances for the treatment of wounds.

So-called civilization diseases, including but not limited to cardiovascular diseases, obesity, diabetes and cancer, are responsible for approximately 40 million deaths worldwide each year, which corresponds to about 70% of all global deaths. It is estimated that cardiovascular diseases and cancer, followed by respiratory system diseases and diabetes, constitute the most frequent causes of mortality among civilization diseases. Currently, more than 2 million people in Poland suffer from diabetes, of whom approximately 25% remain undiagnosed. Forecasts indicate that within the next 15-20 years the number of patients suffering from diabetes in Poland will double. It should be emphasized that the lifetime risk of developing ulceration in diabetic patients is estimated at 12-25%, and the probability of amputation in patients with diabetes is 30-40 times higher than in individuals without diabetes. Moreover, wound healing in diabetic patients is significantly impaired. Therefore, the search for medicinal products of natural origin that could contribute to the improvement of patient health is of particular importance not only from a socio-economic perspective, but also from a clinical standpoint.

According to literature data, the problem of chronic non-healing wounds affects approximately 2 out of 100 individuals, while in more than 10% of patients suffering from diabetes mellitus the development of diabetic foot syndrome and occupational dysfunction may occur. According to forecasts, the global number of individuals affected by diabetes mellitus will reach 333 million by the year 2025. This disease and its complications, including diabetic wounds and ulcers, are regarded as a global epidemic of the 21st century. Therefore, the developed hydrogel dressing containing bioactive substances derived from sheep's milk and camel's milk may have international applicability. Problems related to civilization diseases affect both developed and economically disadvantaged societies. According to the World Health Organization (WHO), low- and middle-income countries, as well as less affluent individuals in all countries, are most severely affected by mortality caused by civilization diseases. This constitutes a vicious circle of risk, in which impoverished populations are increasingly exposed to indirect risk factors for civilization diseases, such as tobacco smoking, unhealthy diet, insufficient physical activity and harmful alcohol consumption, while these diseases, in turn, may play a significant role in pushing individuals and their families into poverty.

As reported by Sezik et al. (2001), sheep milk has been used in traditional medicine for the treatment of hard-to-heal wounds, bites, and purulent skin lesions. Improper diet, recurrent injuries, and diabetes are among the factors that significantly impair the wound healing process. Depending on the size of the wound and the degree of tissue damage, extensive physiological and metabolic changes may occur, leading to complications in the healing process and a reduction in patients' immune resistance (Hrynyk and Neufeld, 2014). Therefore, in the era of the rapidly increasing prevalence of civilization-related diseases, metabolic disorders, various types of allergies, and cancers (e.g. melanoma), it is of particular importance to support the human body with natural products exhibiting specific therapeutic properties. Such products include sheep milk and camel milk due to their rich profile of bioactive substances. Both of these products, in comparison with, for example, cow's milk, contain high levels of insulin, which plays a significant role in the wound healing process. Insulin regulates blood glucose levels and may support the healing of damaged skin by modulating oxidative and inflammatory responses and by stimulating cellular migration. Studies have demonstrated that topical administration of insulin has a significant effect on the healing of diabetic wounds as well as burn wounds, acute wounds, and chronic wounds. In diabetic patients, local injection of insulin at the wound site promotes wound healing by enhancing granulation tissue formation (Zhang and Lv, 2016). However, injection into sensitive tissue is painful for patients and difficult to perform independently, whereas in the case of medical creams containing insulin, the effectiveness of absorption may be limited due to abrasion and removal of the preparation from the wound surface. Therefore, due to ease of application and use, the integration of insulin into hydrogel dressings may represent a breakthrough solution. Studies by Zhao et al. (2017) revealed that the incorporation of insulin and fibroblasts into a hydrogel-based dressing may promote neovascularization and collagen deposition and improve the healing process of diabetic wounds (Zhao et al., 2017). For these reasons, the design and production of hydrogel dressings containing sheep milk and camel milk constitutes a promising approach within the market of specialized dressings intended for the treatment of hard-to-heal wounds.

Lactoferrin (LF) is an iron-binding glycoprotein that supports multiple biological processes associated with wound healing. It exhibits anti-inflammatory activity, directly promotes the formation of granulation tissue, stimulates the proliferation and migration of fibroblasts and keratinocytes, and enhances the synthesis of collagen and hyaluronan. Ovine milk, due to its particularly high lactoferrin content, reduces autoimmune inflammatory processes and exerts a protective effect against bacterial and viral infections (Caboni et al., 2019).

Camel milk is rich in bioactive substances, such as active peptides, lactoferrin (LF), zinc, and mono- and polyunsaturated fatty acids. For this reason, camel milk also exhibits antioxidant, antimicrobial, antidiabetic, and hypocholesterolemic properties. The lactoferrin content in camel milk ranges from 0.2 to 0.9 g/kg, while in sheep milk it ranges from 0.7 to 0.9 g/kg, and in cow's milk it amounts to only 0.02 to 0.5 g/kg (Alichanidis et al., 2016).

In recent years, the use of biomaterials as wound dressings has attracted increasing attention, and hydrogels loaded with bioactive substances demonstrate significant potential in wound treatment as targeted therapeutic delivery systems (Zhao et al., 2017). Camel milk may be used in the treatment of type 1 and type 2 diabetes mellitus, due to its high insulin content-approximately 52-59 units per liter-as well as the presence of insulin-like substances (Abdalla et al., 2015). Proteins derived from sheep milk also constitute a promising source of insulin, antidiabetic peptides, and hypotensive peptides (Iram et al., 2022).

The development of novel wound dressings exhibiting properties that accelerate wound healing has recently been the subject of extensive research. Wang et al. (2022) synthesized a hydrogel containing lactoferrin and lithium-magnesium silicate, which, after injection, effectively supports the wound healing process (Wang et al., 2022). The prospect of producing a hydrogel wound dressing containing natural components such as sheep milk and camel milk, which are rich in bioactive substances, is highly promising. This constitutes a significant opportunity to improve the comfort and effectiveness of treatment for patients suffering from burn wounds or wounds of diabetic origin. Milk fat globules represent an important structural component of milk with both technological and biological significance. The milk fat globule membrane of camel milk exhibits anti-adhesive and antibacterial properties. As reported by Mansour et al. (2015), a cream containing 25% sheep milk fat globules accelerates burn wound healing, reduces the intensity of inflammation, promotes wound contraction, minimizes scar formation, and improves the treatment of burn wounds. Other lipids playing an important biological role include butyric acid, conjugated linoleic acid (CLA), sphingolipids (components of fat globule membranes), and carriers of vitamins A, D, E, and K, as well as carotenoids. The most biologically active CLA isomer is cis-9, trans-11, which inhibits the occurrence and development of skin cancer (Wang and Jones, 2004). Among ruminants, sheep milk contains the highest amount of CLA (Molik et al., 2020). One of the distinctive features of camel milk is the presence of CLA, which exhibits antioxidant and anti-inflammatory activity. Studies conducted by Park et al. (2010) demonstrated that CLA supplementation accelerates skin wound healing by regulating antioxidant and anti-inflammatory functions. This creates the possibility of investigating the topical effect of CLA on the wound healing process.

Hydrogels are characterized by good elasticity, a high water content, and favorable oxygen permeability, which makes them among the most promising materials for wound dressing applications (Zhang et al., 2023). Additionally, nanomaterials exhibit improved biodegradability, biocompatibility, and colloidal stability in the wound healing process and may play a role in promoting healing either through their intrinsic properties or as carriers for other bioactive substances (Zhang et al., 2023). At present, consumers show increasing interest in products of natural origin exhibiting health-promoting properties. Due to the presence of numerous biologically active compounds, sheep milk and camel milk are gaining growing significance. Consequently, the use of milk derived from these animals for the production of hydrogel wound dressings intended for the treatment of hard-to-heal wounds constitutes a particularly promising and innovative solution.

In the state of the art, patent documents relating to hydrogel dressings are also known. For example, U.S. patent application US2024277894 AA discloses a composite antibacterial hydrogel dressing, a method for producing the same, and its use in wound treatment. The dressing comprises a hydrogel capable of retaining a large amount of water, thereby creating a moist environment that supports tissue regeneration. Additionally, the dressing contains antibacterial agents, such as metal nanoparticles (e.g. silver), organic substances, or chemical compounds that eliminate pathogens and prevent infections. The structure of the dressing is multilayered, enabling simultaneous antibacterial activity and effective absorption of wound exudates. The materials used for its production are biodegradable and biocompatible, thereby minimizing the risk of adverse effects. The manufacturing process includes mixing the hydrogel components and cross-linking them, which ensures appropriate elasticity and durability of the final product.

In turn, utility model application DE202023104875 U1 discloses a composite hydrogel wound dressing designed for wound treatment, comprising a hydrogel body adapted to maintain a moist environment, thereby supporting the wound healing process and tissue regeneration. Owing to its composite structure, the dressing may be enriched with components having antibacterial properties, such as metal nanoparticles (e.g. silver), or with substances supporting skin regeneration. The hydrogel is flexible, biocompatible, and configured for effective management of wound exudate, which renders the dressing suitable for the treatment of both chronic and acute wounds. The manufacturing process comprises forming a multilayer material which combines protective, antibacterial, and therapeutic functions within a single dressing structure.

Patent application CN115414523 A discloses a hydrogel wound dressing and a method for producing the same. The disclosed dressing comprises a hydrogel layer configured to provide a moist environment conducive to wound healing. Owing to its hydrogel structure, the dressing is capable of absorbing wound exudates, thereby facilitating wound cleanliness and accelerating tissue regeneration. The method of producing the dressing comprises mixing suitable polymers with water to form a homogeneous composition, which is subsequently subjected to a gelation process under controlled temperature and pH conditions. The hydrogel obtained in this manner is characterized by flexibility, biocompatibility, and the ability to conform to the shape of the wound, thereby improving patient comfort and therapeutic effectiveness. The dressing may be used for the treatment of various types of wounds, including second-degree burns, due to its adhesive and protective properties.

None of the cited documents discloses the use of sheep milk and/or camel milk in hydrogel dressings, nor the use of lyophilisates of sheep milk and/or camel milk.

The objective of the invention was to develop a novel hydrogel dressing comprising bioactive substances. The high content of health-promoting bioactive compounds present in sheep's milk and camel milk determines the unique properties of these raw materials. A further objective of the invention was to develop a method for producing a hydrogel dressing containing bioactive substances.

An important aspect of the developed dressing is that it contains bioactive substances derived from sheep's milk and camel milk, which are obtained in many countries worldwide and therefore do not constitute costly or scarcely available raw materials. Consequently, the production technology of the developed dressing is not expensive and may be implemented in any country.

The produced hydrogel dressing functionalized with bioactive substances of sheep's milk and camel milk constitutes a product of natural origin. Another objective of the invention was the use of the dressing for wound treatment.

The developed hydrogel dressing may represent a significant factor in the treatment of difficult-to-heal wounds, including diabetic wounds, due to the rich content of bioactive substances such as insulin, lactoferrin, orotic acid, and conjugated linoleic acid. These substances are of particular importance in the wound-healing process owing to their properties, including anti-inflammatory activity, stimulation of cell proliferation and migration, and stimulation of collagen synthesis. The developed biomaterials based on natural milk-derived bioactive substances may significantly improve both the effectiveness and comfort of treatment of patients with skin disorders, with particular emphasis on diabetic patients. The developed dressing constitutes an innovative yet cost-effective technological solution applicable in European Union countries and beyond.

The essence of the invention is a hydrogel dressing comprising at least one layer of a hydrogel matrix with at least one bioactive substance, characterized in that said hydrogel matrix comprises from 4 to 8 wt.% of an aqueous solution of polyvinyl alcohol (PVA) and 4 wt.% of an aqueous solution of sodium tetraborate (borax), and wherein the bioactive substance is sheep milk in an amount of from 5 to 20 wt.% and/or camel milk in an amount of from 2.5 to 15 wt.%.

The hydrogel exhibits an appropriate structure enabling free diffusion of bioactive particles within the gel matrix. Sodium tetraborate is the most suitable crosslinking agent for polyvinyl alcohol (PVA); it is a safe crosslinker and inherently exhibits mild bactericidal properties. It effectively stabilizes the hydrogel scaffold and enables the production of reproducible hydrogels.

Preferably, in the hydrogel dressing according to the invention, the hydrogel matrix comprises 4 wt.% of an aqueous solution of polyvinyl alcohol (PVA).

Alginate-based hydrogels were also tested; however, in this case it was difficult to achieve reproducibility and stability of the hydrogel, as such structures underwent rapid biological degradation.

Preferably, in the hydrogel dressing according to the invention, the sheep milk and/or camel milk is present in the form of a lyophilisate.

Lyophilization is intended to preserve the bioactive components contained in the milk without causing damage thereto. The application of thermal processing to milk results in thermal degradation of the most sensitive molecules. The lyophilisate additionally enables storage of milk in a safe and stable powder form, which is not possible when water is present in the milk, as biodegradation processes and decomposition caused by bacteria and fungi may then occur. Another method tested for isolating bioactive substances from milk was centrifugation at a speed of 35,000 revolutions per minute. However, the products obtained thereby were unsuitable for use in hydrogel dressings due to their water content. Lyophilization has proven to be decidedly the most advantageous method, as it provides a reproducible product that consistently retains the full composition of milk, including its bioactive substances, while being devoid of water. Centrifugation is burdened with risk, since during decantation of the supernatant from the solid fraction, residual water remains, and the centrifuged powder is moist. This may lead to a reduced shelf life of the powder obtained by centrifugation.

It is further advantageous when the hydrogel dressing according to the invention comprises three layers, wherein: the inner layer closest to the wound contains lyophilisate of sheep milk in an amount of from 10 to 20 wt.% and lyophilisate of camel milk in an amount of from 10 to 15 wt.%; the intermediate layer contains from 5 to 15 wt.% of lyophilisate of sheep milk and from 2.5 to 10 wt.% of lyophilisate of camel milk; and the outer layer contains exclusively the hydrogel matrix.

Particularly preferably, the hydrogel dressing according to the invention comprises three layers, wherein: the inner layer, located closest to the wound, contains 15 wt.% of lyophilisate of sheep milk and 10 wt.% of lyophilisate of camel milk; the intermediate layer contains 5 wt.% of lyophilisate of sheep milk and 5 wt.% of lyophilisate of camel milk; and the outer layer contains exclusively the hydrogel matrix.

In another aspect, the invention also relates to a method of producing a hydrogel dressing comprising the following steps:
a) preparing a bioactive substance in the form of a lyophilisate obtained from sheep milk and/or a lyophilisate obtained from camel milk, at a temperature in the range from -90°C to -60°C;
b) preparing a hydrogel matrix by mixing an aqueous solution of polyvinyl alcohol prepared at a temperature in the range from 60°C to 80°C and mixing an aqueous solution of sodium tetraborate prepared at a temperature in the range from 35°C to 45°C, and subsequently adding dropwise the aqueous solution of sodium tetraborate to the aqueous solution of polyvinyl alcohol and mixing until a gel is obtained;
c) adding the bioactive substance to the hydrogel matrix in the form of sheep milk in an amount from 5 to 20 wt.% and/or camel milk in an amount from 2.5 to 15 wt.%.

Preferably, the bioactive substance in step a) of the method for producing the hydrogel dressing according to the invention is prepared at a temperature of -80 °C.

Equally preferably, the borax solution in step b) of the method for producing the hydrogel dressing according to the invention is prepared at a temperature of 40 °C.

The subject matter of the invention is illustrated in exemplary embodiments that do not limit the scope thereof and in the drawing, in which:
Fig. 1 illustrates a method of preparing a PVA solution,
Fig. 2 illustrates a method of preparing a borax solution.
Fig. 3 illustrates a method of preparing a 4/8% PVA hydrogel,
Fig. 4 shows a DSC curve for a dressing layer containing 15 wt.% of sheep milk,
Fig. 5 shows a DSC curve for a PVA/borax hydrogel matrix,
Fig. 6 shows a DSC curve for a PVA/borax sample with the addition of 10 wt.% of lyophilisate of camel milk,
Fig. 7 shows TG and DTG curves for the PVA/borax matrix,
Fig. 8 shows TG and DTG curves for a layer consisting solely of sheep milk (SM),
Fig. 9 shows TG and DTG curves for a hydrogel matrix with the addition of camel milk (CM)
Fig. 10 illustrates the course of changes in the values of E' and E" of the pure hydrogel matrix as a function of frequency,
Fig. 11 illustrates the course of changes in the values of E' and E" of the CM dressing (camel milk) as a function of frequency,
Fig. 12 illustrates the course of changes in the values of E' and E" of the 3MEB dressing (sheep milk) as a function of frequency.
Fig. 13 shows an image of the CM 10B dressing sample prior to the compressive strength test,
Fig. 14 shows an image of the CM 10E dressing sample prior to the compressive strength test,
Fig. 15 presents a compression curve of a sample made of PVA/borax hydrogel,
Fig. 16 presents a compression curve of a sample made of CM 5% hydrogel,
Fig. 17 presents a compression curve of a sample made of CM 10% hydrogel,
Fig. 18 presents a compression curve of a sample made of SM 5% hydrogel,
Fig. 19 presents a compression curve of a sample made of SM 10% hydrogel,
Fig. 20 presents a compression curve of a sample made of SM 10% + CM 15% hydrogel,

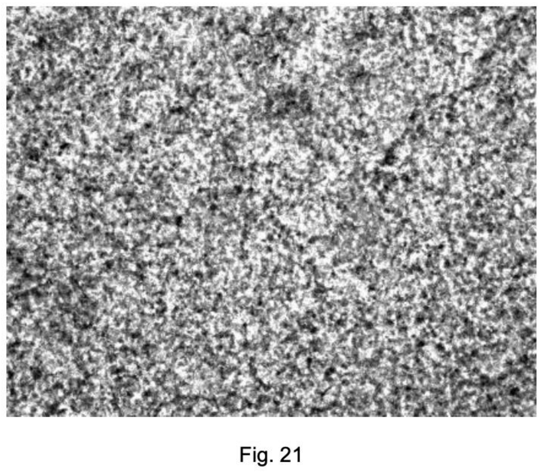

Fig. 21 shows an image of the dressing in visible light (Axiocam503, Zeiss, magnification 5×).

In the exemplary embodiment described below, a detailed description of the dressing together with its characteristics, the method of its manufacture, and the results of studies concerning the safety of its use and its effect on the skin are presented.

In the exemplary embodiment, the dressing was prepared in three stages. In the first stage, the active substance was prepared; in the second stage, the hydrogel matrix was prepared; and in the third stage, the dressing was formed.

Preparation of the active substance: Camel milk and sheep milk were subjected to lyophilization at a temperature of -80 °C (tested temperature range from -95 °C to -60 °C). It was observed that, within the applied temperature range, the bioactive substances contained in the milk were not degraded. This condition is crucial in the context of entrapping the active substances derived from sheep milk and camel milk within the hydrogel structure of the dressing.

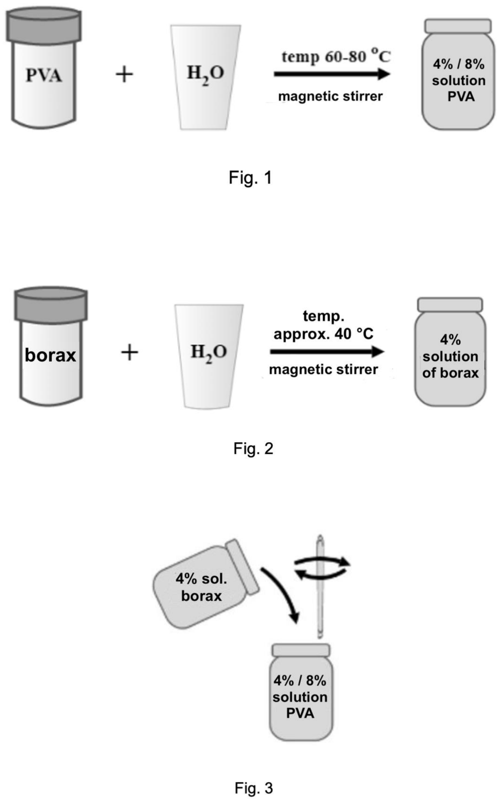

Preparation of the hydrogel matrix: The hydrogel matrix was obtained from polyvinyl alcohol (PVA) and sodium tetraborate (borax) (Figs. 1-3). Preparation of the PVA solution comprises dissolving pure PVA powder in water at a water temperature of 80 °C under intensive stirring. Preparation of the borax solution comprises dissolving borax in water at a temperature of 40 °C under intensive stirring. The borax solution is then dropwise added to the PVA solution. During the dropwise addition, the PVA solution is subjected to intensive stirring using a mechanical stirrer.

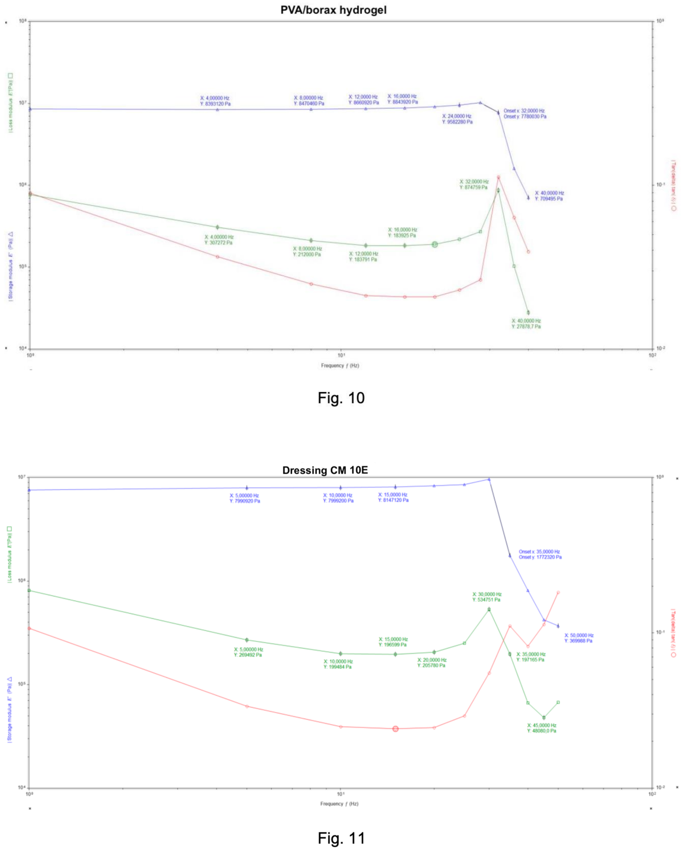

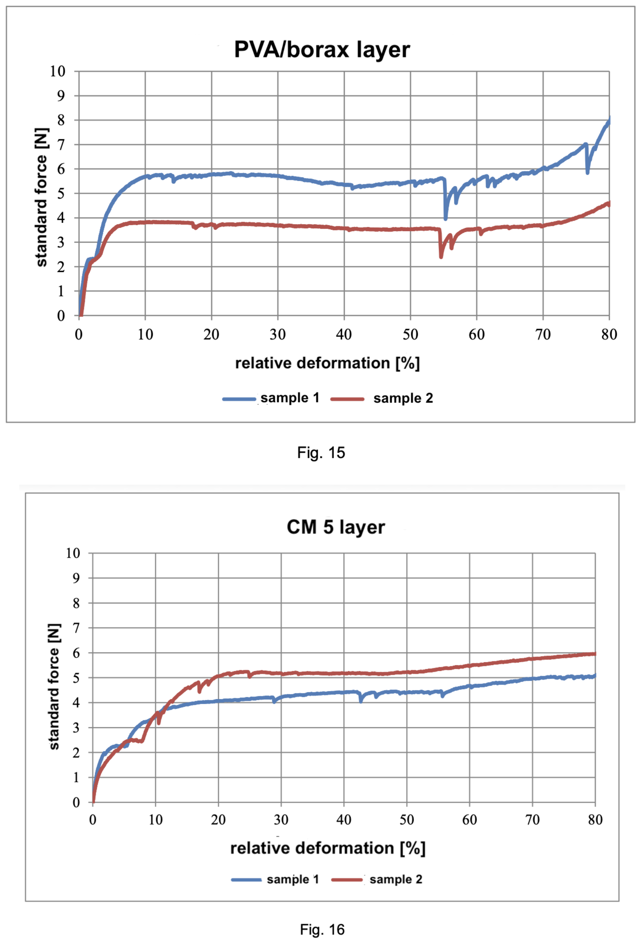

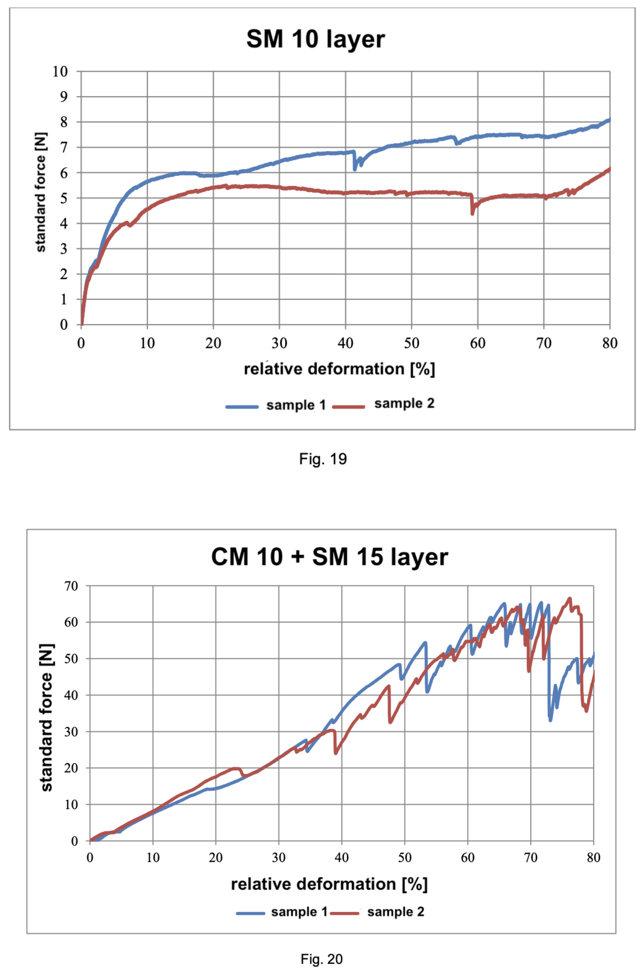

Selection of the optimal concentration was based on DMA tests (results illustrated in Figs. 10-13), which verified mechanical resistance in the vibration frequency range from 1 Hz to 100 Hz, as well as on compressive mechanical strength tests (results illustrated in Figs. 15-20).

Preparation of a three-layer hydrogel dressing: The milk lyophilisate is added to polyvinyl alcohol (PVA) dissolved in water, and the mixture is stirred for 3-5 minutes after the addition of the lyophilisate. Subsequently, sodium tetraborate is added to the PVA containing the lyophilisate and the mixture is stirred again until a gel is obtained. The three-layer hydrogel dressing comprises three gradient layers:
a) an inner layer (closest to the skin) containing 20 wt.% of lyophilisate of sheep milk and 15 wt.% of lyophilisate of camel milk (the most optimal content of lyophilisate of sheep milk being 15 wt.% and of lyophilisate of camel milk being 10 wt.%);
b) a middle layer, which is the most optimal layer, containing from 5 to 10 wt.% of lyophilisate of sheep milk and from 5 to 10 wt.% of lyophilisate of camel milk;
c) an outer layer constituting a protective layer, which does not contain sheep milk or camel milk.

Within the scope of the conducted studies, various formulations and compositions of hydrogel wound dressings were tested (the results are presented in Table 1). The conclusions drawn from the conducted studies are as follows: (1) Below 2.5 wt.% camel milk (CM) and 5 wt.% sheep milk (SM), no bioactive concentration effect is observed, as the content of bioactive substances is insufficient and the resulting effect is unsatisfactory, in particular with respect to the diffusion of bioactive substances from the lyophilisate of milk into the wound; (2) Above 15 wt.% CM and 20 wt.% SM, difficulties occur during the synthesis process, wherein the hydrogel begins to phase-separate and the lyophilisate is released together with water from the hydrogel matrix; it is not possible to bind a higher amount of lyophilisate within the hydrogel structure.

The obtained dressings were examined using the following methods: thermogravimetric analysis (TG), differential scanning calorimetry (DSC), dynamic mechanical analysis (DMA), compressive strength testing, optical microscopy, and conductometric measurements determining the ion release rate from individual layers, serving as an indirect determination of insulin diffusion.

**Table 1. Tested formulations and compositions of hydrogel dressings (CM - camel milk, SM - sheep milk)**

| | | | |
|---|---|---|---|
| PVA/Borax or CM 0, SM 0 | 3 ml of a 4 % PVA solution + 1 ml of a 4 % borax solution | | |
| CM 1 | 3 ml of a 4 % PVA solution + 1 ml of a 4 % borax solution + 0,003 g CM | | |
| CM 2,5 | 3 ml of a 4 % PVA solution + 1 ml of a 4 % borax solution + 0,075 g CM | | |
| CM 5 | 3 ml of a 4 % PVA solution + 1 ml of a 4 % borax solution + 0,15 g CM | | |
| CM 10 | 3 ml of a 4 % PVA solution + 1 ml of a 4 % borax solution + 0,3 g CM | | |
| CM 15 | 3 ml of a 4 % PVA solution + 1 ml of a 4 % borax solution+ 0,45 g CM | | |
| SM 15 | 3 ml of a 4 % PVA solution + 1 ml of a 4 % borax solution + 0,45 g SM | | |
| SM 10 | 3 ml of a 4 % PVA solution + 1 ml of a 4 % borax solution + 0,3 g SM | | |
| SM 5 | 3 ml of a 4 % PVA solution + 1 ml of a 4 % borax solution + 0,15 g SM | | |

| Type of a three-layer dressing | Outer layer | Intermediate layer | Inner layer (intended for contact with the skin) |
|---|---|---|---|
| | Volumes and masses of the substances used | | |
| Extremely low | 3 ml of a 4 % PVA solution | 3 ml of a 4 % PVA solution | 3 ml of a 4 % PVA solution |
| | 1 ml of a 4 % borax solution | 1 ml of a 4 % borax solution | 1 ml of a 4 % borax solution |
| | 0% CM | 2,5% CM | 5% CM |
| | 0% SM | 5% SM | 5% SM |
| Extremely high | 3 ml of a 4 % PVA solution | 3 ml of a 4 % PVA solution | 3 ml of a 4 % PVA solution |
| | 1 ml of a 4 % borax solution | 1 ml of a 4 % borax solution | 1 ml of a 4 % borax solution |
| | 5% CM | 10% CM | 15% CM |
| | 5% SM | 15% SM | 20% SM |
| Optimal composition | 3 ml of a 4 % PVA solution | 3 ml of a 4 % PVA solution | 3 ml of a 4 % PVA solution |
| | 1 ml of a 4 % borax solution | 1 ml of a 4 % borax solution | 1 ml of a 4 % borax solution |
| | 0% CM | 5% CM | 10% CM |
| | 0% SM | 5% SM | 15% SM |

### Results of DSC and TG studies

By means of differential scanning calorimetry (DSC), DSC curves of various types of layers of the produced hydrogel materials were obtained. The experiment was conducted on substances such as a PVA/borax hydrogel, which constituted the reference material, as well as on individual layers of the hydrogel dressing composed of a PVA/borax matrix and the active substances used.

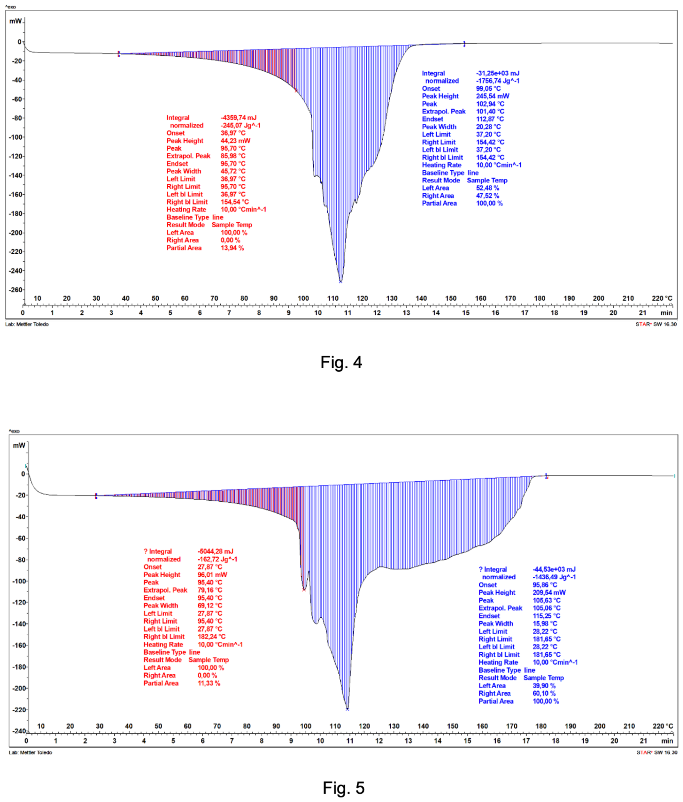

The obtained plots are presented below (Fig. 4-6).

**Table 2. Values read from the obtained TG/DTG curves for the individual hydrogels**

| Type of the tested sample" | Values obtained from the DSC analysis | | | | |
|---|---|---|---|---|---|
| | Evaporation of water up to 100 °C | | Decomposition of the sample up to 220 °C | | |
| | Onset of water evaporation [°C] | Evaporation energy [J/g] | End of water evaporation [°C] | Evaporation energy [J/g] | Number of peaks |
| PVA/borax | 30,12 | 170,54 | 178,79 | 1424,07 | 4 |
| C milk | 34,58 | 344,24 (↑ 101,85%) | 144,23 | 1761,41 (↑ 23,69%) | 3 |
| S milk | 36,97 | 45,07 (↑50,61%) | 154,42 | 1756,74 (↑22,30%) | 8 |

By comparing the evaporation energy values up to 100 °C for the examined materials, it can be concluded that the lowest energy required to evaporate 1 g of mass was exhibited by the basic PVA/borax hydrogel matrix. Each addition of a substance increased the evaporation energy of water up to 100 °C and thereby strengthened the structure of the formed hydrogel. Over the entire temperature range of the conducted analysis, it can be observed that each addition of an active substance to the pure PVA/borax hydrogel matrix resulted in a decrease in the temperature corresponding to the end of water evaporation from the hydrogel, which may indicate a loosening of the matrix structure or an increase in the content of free water. However, when considering the energies required for water evaporation from the matrix up to 220 °C, an increase in these energies is observed in most cases. The increase in energy indicates an increase in the compactness of the hydrogel structure. The only exception is the hydrogel containing 10% camel milk and 15% sheep milk (10 CM, 15 SM), for which a decrease in the water evaporation energy was observed; this hydrogel contained lower amounts of both free and bound water within its structure, as demonstrated by TG analysis. Nevertheless, wound dressings operate at temperatures close to human body temperature. The analysis demonstrated that each addition of an active substance increases the evaporation energy, resulting in more difficult water evaporation from the hydrogel. Owing to this relationship, such dressings are capable of maintaining a moist environment essential for tissue regeneration processes.

### Results of TG Studies

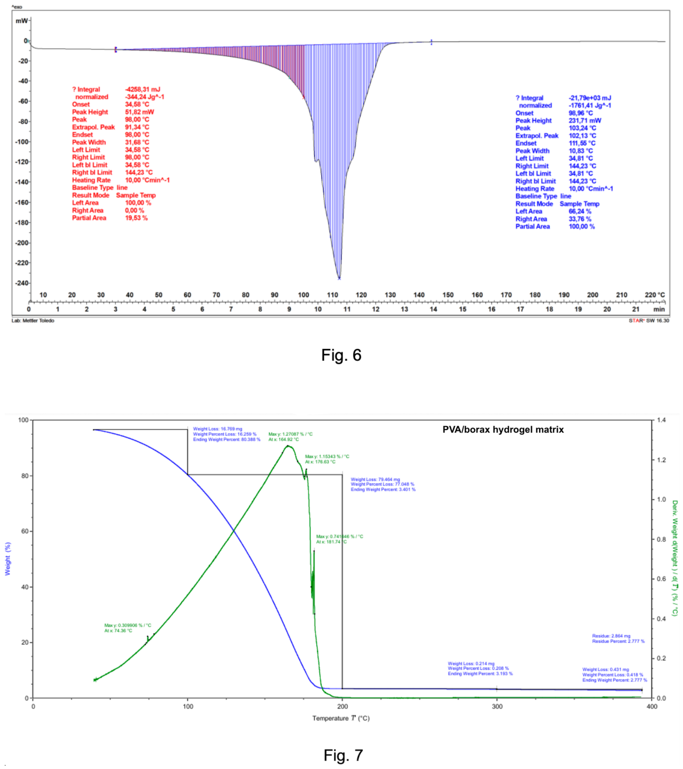

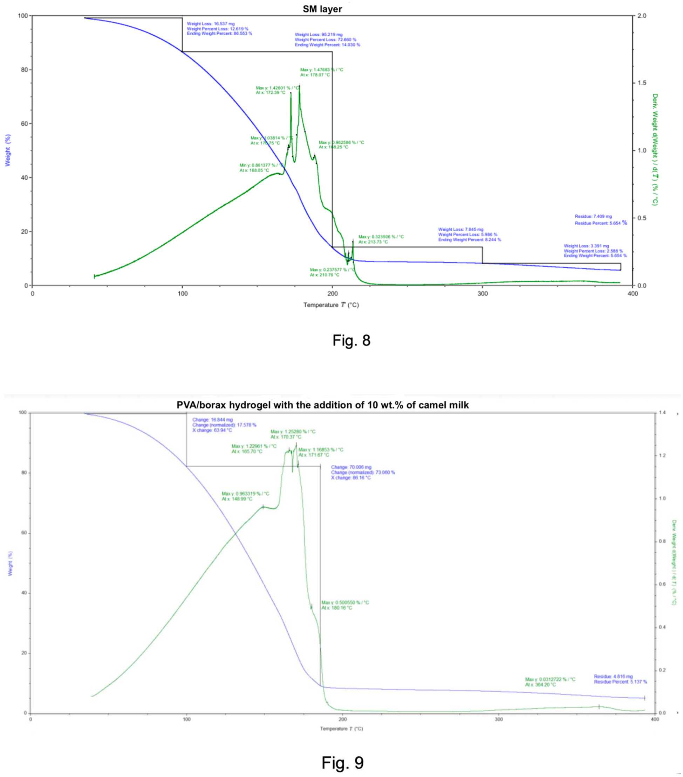

The TG and DTG curves obtained for the produced pure hydrogel matrix as well as for the matrices enriched with selected additives (sheep milk and camel milk) are presented in Figures 7-9.

The PVA/borax hydrogel, serving as a matrix at the interface between the solid state and water, contains a high proportion of free water, amounting to approximately 17%, incorporated within its volume. The matrix additionally contains strongly bound water, the evaporation of which is completed at temperatures of approximately 200 °C. Despite the relatively low intrinsic stability of borax, the substances used for the production of the base matrix allow for the formation of a stable hydrogel structure with a high content of bound water. In comparison with the pure hydrogel, the content of free water in the matrix enriched with lyophilisate of camel milk is higher by only 1 percentage point. The content of strongly bound water, however, differs by approximately 4 percentage points, which allows it to be concluded that the addition of lyophilisate of camel milk reinforces the structure of the hydrogel.

The information contained in the results within the temperature range of 0-100°C illustrates the amount of free water present in the hydrogel structure. The PVA/borax matrix as well as the EE layer exhibit the highest content of free water, with values ranging from 17-20%. The addition of a bioactive substance to the hydrogel significantly reduces the amount of free water evaporated from the structure by approximately 6-8 percentage points, which indicates a strengthening of the hydrogel structure due to the incorporation of active additives into its matrix. Hydrogel matrices are also characterized by the presence of strongly bound water within their structure, the evaporation of which terminates at approximately 200°C. Within this temperature range, a reduced evaporation of water can also be observed. The incorporation of additives resulted in differences in the content of strongly bound water within the hydrogel structure of up to 51 percentage points, with such a result being achieved in the MEE layer. The percentage of retained final mass at a temperature of 400°C is the highest for the SM layer.

### Results of DMA Tests

The conducted DMA tests were aimed at simulating the conditions under which the dressing operates when applied to a patient's skin. The test temperature was kept constant at 36 °C, while the variable parameter was the frequency. Since the dressing operates together with the skin to which it is applied and must withstand compressive forces, this test enabled evaluation of its ability to transfer stresses as well as determination of critical frequency values leading to hydrogel failure. The analysis examined changes in the storage modulus E' (responsible for stress transfer in solid bodies) and changes in the loss modulus E" (responsible for energy dissipation). The obtained results are shown in Figures 10-12, corresponding to the pure hydrogel matrix and the target dressings containing milk-based additives.

In the case of the pure hydrogel matrix, the study demonstrated that the value of the storage modulus E' remains constant within the frequency range of 1-32 Hz and oscillates around a value of 8,500,000 Pa. A decrease in the value of E', and thus a deterioration of the mechanical properties of the PVA/borax hydrogel, is observed only after exceeding the operating frequency of 32 Hz. Similar relationships are observed for the dressing comprising an addition of camel milk. The obtained values of the storage modulus E' indicate that the most favourable results, i.e. stability of properties over the widest frequency range, were achieved for the dressing comprising an addition of sheep milk.

The produced dressing exhibits adequate static and dynamic mechanical strength within the vibration frequency range, enabling the transfer of stresses that may occur and do occur during the daily use of dressings of this type.

### Results of mechanical testing

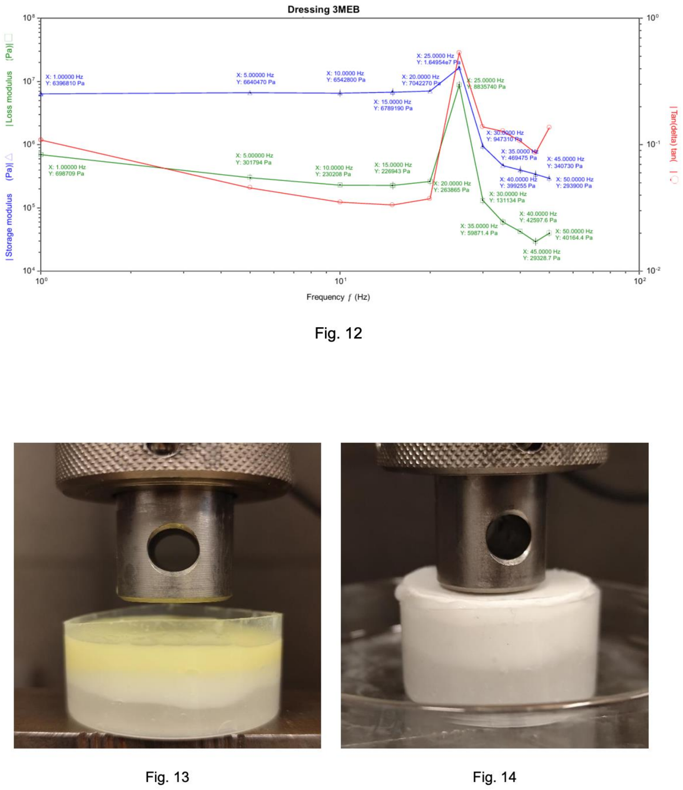

During the compression tests of the produced dressings, individual layers of selected dressings as well as complete hydrogel dressings were examined separately. Each test was performed in two repetitions. The appearance of representative samples subjected to the compressive strength test is shown in the figures. The shape of the tested samples was constrained on all sides, and during the test the compressive force was measured until a relative shortening of 80% was achieved. The curves illustrating the relationship between the applied compressive force (expressed in newtons, N) and the sample shortening (expressed as a percentage) are presented for individual samples in the respective graphs (Figures 13-20).

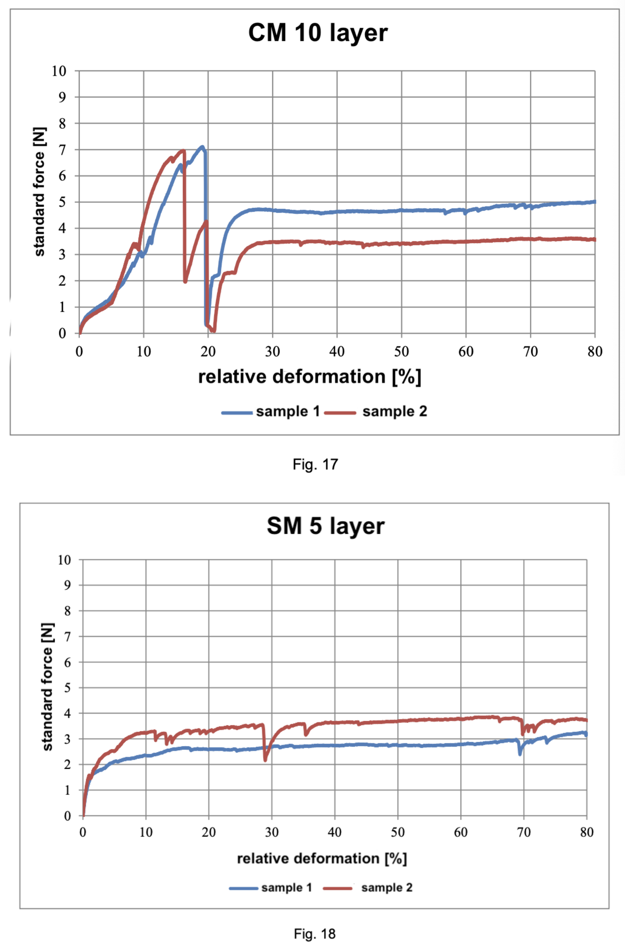

In the case of the pure PVA/borax hydrogel matrix, the compression curve of which is shown in Fig. 13, the hydrogel withstands a compressive force of approximately 6 N while reducing its dimension by 10%, which corresponds to a shortening of about 1.7 mm. This resistance is maintained over a further displacement of 12 mm, resulting in a total sample shortening of 80% relative to the initial height. Minor fluctuations observed in this curve, as well as in subsequent curves, indicate the presence of air trapped within the hydrogel volume and its release from the structure during compression of the material. The addition of 1% camel milk, relative to the volume of PVA used in the preparation of the matrix, caused the hydrogel to reach a 10% reduction in height at a compressive force lower by approximately 2 N. Under further loading, the sample exhibits a slight strengthening effect. Based on these results, it can be concluded that despite the relatively small amount of active substance added to the dispersed phase, the hydrogel becomes more plastic and deforms under lower applied force. A significant change in mechanical properties is observed for the hydrogel containing 10% of lyophilisate of camel milk, the test results of which are presented in Fig. 17. The highest resistance exhibited by the hydrogel was observed at a relative shortening of 15-20%, reaching a force value of approximately 7 N. The presence of a higher amount of milk lyophilisate within the hydrogel structure favourably affects its ability to transfer compressive loads, which are the dominant forces encountered by wound dressings during use. This constitutes a beneficial effect resulting from the incorporation of milk lyophilisate into the hydrogel scaffold.

In a subsequent stage, analyses were performed using in vitro techniques, employing normal human skin cells. The use of keratinocyte-based studies enables the assessment, under laboratory conditions, of the safety of use of a given substance and its effect on the skin. Keratinocytes are epithelial cells present in all layers of the epidermis and constitute approximately 95% of epidermal cells. The aim of the conducted analyses was to observe the behaviour of epidermal cells, namely keratinocytes, during wound healing processes upon application of the developed dressing. Wound healing is characterized by three overlapping phases: inflammation, proliferation, and remodelling. Among these phases, the proliferation phase is critical for the wound healing process; therefore, this phase was specifically analysed in the cellular studies

### Safety of use of the hydrogel dressing according to the invention and its effect on the skin.

Two types of studies were conducted: (1) seeding of cells directly onto the three-layer hydrogel dressings; (2) cultivation of cells in conditioned medium (CM).

In both experimental variants, the manufactured dressings were sterilized by exposure to ultraviolet (UV) radiation for 30 minutes at room temperature. Each type of three-layer hydrogel dressing was prepared in three replicates.

Human primary keratinocytes, commercially available, were used in the studies. Cell cultures were maintained in complete Dulbecco's Modified Eagle's Medium - high glucose (DMEM, No. 1145, Sigma Aldrich), supplemented with 10% FBS (Fetal Bovine Serum), thereby providing optimal conditions for in vitro cultivation. In addition, antibiotics (penicillin and streptomycin) were added to the culture medium in order to ensure sterility of the entire cell culture. The cultures were maintained in a CO₂-controlled incubator (5% CO₂ / 95% air) at 37°C (which are standard conditions for the cultivation of human cells).

### Direct seeding of cells onto the dressing

Prior to seeding, the cells were stained with the CellTracker^{™} Green CMFDA fluorescent dye (Invitrogen, USA), which enabled observation of cell number, morphology, and spatial distribution using a fluorescence microscope by excitation of fluorescence with blue light (λₓ = 492 nm; λₘ = 517 nm). The incubation with the dye was carried out for 30 minutes at a temperature of 37 °C in an incubator, in accordance with the manufacturer's instructions.

In the case of the first type of conducted experiment, the cells were seeded onto the three-layer hydrogel dressing so as to be in direct contact with the layer intended to be positioned adjacent to the patient's skin. Into each culture well containing the dressing, 200 µL of a cell suspension in complete culture medium was pipetted, corresponding to 10,000 cells per well. The cells together with the dressing were incubated for 48 hours under standard conditions (37 °C, 5% CO₂, 95% oxygen).

However, due to the opacity of the dressing, it was not possible to assess cell morphology and cell number using fluorescence microscopy, and therefore these measurements did not provide the expected or meaningful results.

Therefore, in the subsequent stage, following cell seeding and staining with CellTracker Green CMFDA, a multiwell plate reader was employed for quantitative fluorescence measurement. However, due to the low sensitivity of this method, it was excluded from further analyses and served only to confirm the presence of cells on the dressing. After 24 hours, the highest fluorescence intensity, corresponding proportionally to the number of viable cells, was recorded for the control dressing. In the case of the dressing containing camel milk and vitamin E (CME), the highest cell viability was observed at a vitamin E concentration of 5%, whereas the lowest viability was observed at a concentration of 2.5% (Table 3). For the dressing containing vitamin B (CMB), an increase in cell viability was observed with increasing concentrations of the vitamin (Table 4). However, the obtained results were not considered satisfactory, as no statistically significant differences were observed between the control dressing, consisting solely of the pure PVA/borax hydrogel matrix, and the enriched dressings.

**Table 3. Fluorescence intensity measurement values for cells cultured with dressings after 24 h of incubation using the CellTracker^{™} Green CMFDA dye.**

| Cell fluorescence intensity after 24 h culture on the dressing [a.u.] | | | | |
|---|---|---|---|---|
| Type of dressing | PVA/Borax | CMB (2,5) | CMB (5) | CMB (10) |
| Well 1 | 9,90 | 7,43 | 9,14 | 8,05 |
| Well 2 | 11,55 | 9,18 | 10,84 | 7,48 |
| Well 3 | 12,75 | 7,10 | 10,33 | 8,44 |
| Average value | 11,40 | 7,90 | 10,10 | 8,14 |
| Standard deviation | 1,43 | 1,12 | 0,87 | 0,57 |

**Table 4. Values of fluorescence intensity measurements for cells cultured with dressings after 24 h of incubation using the CellTracker Green CMFDA dye.**

| Cell fluorescence intensity after 24 h culture on the dressing [a.u.] | | | | |
|---|---|---|---|---|
| Type of dressing | PVA/Borax | CMB (2,5) | CMB (5) | CMB (10) |
| Well 1 | 9,90 | 9,24 | 8,42 | 9,99 |
| Well 2 | 2 9,99 | 9,71 | 9,26 | 11,45 |
| Well 3 | 12,75 | 8,15 | 12,65 | 9,98 |
| Average value | 11,40 | 9,03 | 10,11 | 10,48 |
| Standard deviation | 1,43 | 0,80 | 2,24 | 0,84 |

In a subsequent stage, following the seeding of cells onto the dressings as described above, the CellTiter-Glo^{®} Luminescent Cell Viability Assay (Promega) was applied, which determines the number of viable cells based on the quantitative measurement of adenosine triphosphate (ATP) present. The assay requires only the direct addition of the reagent to the cells followed by measurement. After 48 hours, luminescence measurements were performed, the results of which are presented in Table 5. In contrast to the previously applied test, after 48 hours the lowest number of viable cells was observed for the pure PVA/borax hydrogel matrix. This indicates that each modifier addition in the form of an active substance had a positive effect on cell viability.

**Table 5. Cell viability after 48 h of incubation with the dressing based on quantitative ATP measurement**

| Luminescence intensity after culturing cells on the dressing for 48 h | | | | |
|---|---|---|---|---|
| Type of dressing | PVA/Borax | CMB 2,5 | CMB 5 | CMB 10 |
| Well 1 | 1101 | 7728 | 6919 | 10896 |
| Well 2 | 908 | 6614 | 8873 | 10680 |
| Well 3 | 1349 | 4505 | 8616 | 12770 |
| Average value | 1119 | 6282 | 8136 | 11449 |
| Standard deviation | 221 | 1637 | 1062 | 1149 |

### Culture of cells in conditioned medium

The second type of experiment conducted using keratinocytes involved culturing the cells in a conditioned medium. The purpose of this study was to determine the effect of substances released from the hydrogel matrix on human keratinocytes. The conditioned medium was obtained by incubating the dressings in DMEM culture medium for 24 hours at a temperature of 4 °C, such that the layer of the dressing intended to be in contact with the patient's skin was in direct contact with the medium (spent medium). Subsequently, the spent medium was collected from above the dressing and mixed with complete culture medium in various volume ratios. Keratinocytes were seeded onto 96-well culture plates at a density of 4,000 cells per well and cultured in the conditioned medium for 24 hours. Cell proliferation was assessed using a commercially available fluorometric assay for cell proliferation analysis, PrestoBlue (Thermo Fisher Scientific). For the 1:1 ratio, 50% v/v of spent medium was mixed with 50% v/v of complete culture medium. For the 1:4 ratio, 20% v/v of spent medium was mixed with 80% v/v of complete culture medium, and for the 1:19 ratio, 5% v/v of spent medium was mixed with 95% v/v of complete culture medium. Table 6 presents the results obtained for the 1:1 ratio. Table 7 presents the results obtained for the 1:4 ratio, in which an increase in cell proliferation was observed compared to the control (PVA/borax matrix).

**Table 6. Cell viability after 24 h incubation in conditioned medium at a 1:1 ratio, obtained on the basis of luminescence intensity measurements using the PrestoBlue dye.**

| Cell fluorescence intensity after incubation (24 h) with conditioned medium at a 1:1 ratio [a.u.] | | | | |
|---|---|---|---|---|
| Type of dressing | PVA/Borax | CME 2,5 | CME 5 | CME 10 |
| Well 1 | 72,92 | 114,1 | 113,8 | 114,6 |
| Well 2 | 64,39 | 100,1 | 101,6 | 134,2 |
| Well 3 | 65,27 | 103,0 | 107,2 | 105,9 |
| Average value | 67,5 | 105,7 | 107,5 | 118,2 |
| Standard deviation | 4,7 | 7,4 | 6,1 | 14,5 |

**Table 7. Cell viability after 24 h of incubation in conditioned medium at a 1:4 ratio, obtained on the basis of luminescence intensity measurements using the PrestoBlue reagent.**

| Cell fluorescence intensity after incubation (24 h) with conditioned medium at a 1:1 ratio [a.u.]" | | | | |
|---|---|---|---|---|
| Type of dressing | PVA/Borax | CME 2,5 | CME 5 | CME 10 |
| Well 1 | 105,5 | 116,1 | 113,2 | 125,5 |
| Well 2 | 84,29 | 117,1 | 101,8 | 107,2 |
| Well 3 | 89,08 | 101,9 | 120,7 | 108,4 |
| Average value | *93,0* | *111,7* | *111,9* | *113,7* |
| Standard deviation | *11,1* | *8,5* | *9,5* | *10,2* |

Table 8 presents the results obtained for the 1:19 ratio, wherein the measurement was performed after 48 hours for sheep milk (SM), demonstrating a significant and high increase in cell proliferation compared to the control (PVA/borax).

**Table 8. Cell viability after 48 h incubation in conditioned medium at a 1:19 ratio, determined on the basis of luminescence intensity measurements using the PrestoBlue dye.**

| Type of dressing | PVA/Borax | SM |
|---|---|---|
| Well 1 | 7816 | 12303 |
| Well 2 | 8519 | 13506 |
| Well 3 | 7895 | 14033 |
| Well 4 | 8317 | 14880 |
| Well 5 | 7384 | 14458 |
| Well 6 | 7868 | 12994 |
| Average value | 7967 | 13696 |
| Standard deviation | 401 | 956 |

The contemporary concept of topical treatment of chronic and hard-to-heal wounds, in accordance with the strategy developed by the European Wound Management Association (EWMA) known as TIME (T - tissue management, I - infection and inflammation control, M - moisture balance, E - epithelial advancement), comprises the following sequential steps: wound debridement, control of infection and inflammation within the wound bed, maintenance of appropriate moisture balance, and stimulation of epithelialization. Ensuring adequate moisture conditions within the wound bed and supporting endogenous tissue self-repair processes constitute key objectives in which modern biologically active dressings play a crucial role. Conventional methods based on dry or drying gauze dressings are increasingly being abandoned. A dry compress does not provide any function beyond external wound coverage and protection. Moreover, replacement of a dry dressing may be accompanied by trauma to newly formed tissue structures, which significantly prolongs the overall healing time. Alternative therapeutic approaches currently used in clinical practice include preparations containing cellular growth factors, negative pressure wound therapy (NPWT), and antibacterial dressings incorporating silver. The developed modern, biologically active dressing functionalized with active substances derived from sheep milk and camel milk exhibits characteristics enabling effective competition with currently available commercial products. To date, no comparable technological solution has been identified on the market. Owing to the rich profile of bioactive compounds present in sheep milk and camel milk, the dressing not only provides external wound protection but also actively stimulates natural tissue repair processes, ensures an optimal wound healing environment, and protects the wound interior against the development of infection.

### Literature cited:

**1.** Abdalla, E. et al. Milk Production Potential in Maghrebi She-Camels. Small Rumin. Res. 2015, 123, 129-135. https://doi.org/10.1016/j.smallrumres.2014.11.004.
**2.** Alichanidis, E. et al. Chapter 5-Composition and Properties of Non-Cow Milk and Products. In Non-Bovine Milk and Milk Products; Tsakalidou, E., Papadimitriou, K., Eds.; Academic Press: San Diego, CA, USA, 2016; pp. 81- 116, ISBN 978-0-12-803361-6.
**3.** Caboni, P. et al. A Metabolomics Comparison between Sheep's and Goat's Milk. Food Res. Int. 2019, 119, 869-875. https://doi.org/10.1016/j.foodres.2018.10.071.
**4.** Iram, D. et al. In Silico Identification of Antidiabetic and Hypotensive Potential Bioactive Peptides from the Sheep Milk Proteins-a Molecular Docking Study. J. Food Biochem. 2022, 46, e14137. https://doi.org/10.1111/jfbc.14137.
**5.** Mansour, N. et al. Clinical study on the effect of the sheep milk fat globules on deep second degree burns on pigs. Assiut Vet. Med. J. 2015, 61, 1-14. https://doi.org/10.21608/avmj.2015.170258.
**6.** Molik E and Kordeczka K. 2020.: HEALTH BENEFITS OF SHEEP'S MILK FAT AND ITS IMPACT ON HUMAN HEALTH. Wiadomości Zootechniczne, R. LVIII (2020), 3-4: 38-42.
**7.** Park, N.-Y. et al. Effect of Dietary Conjugated Linoleic Acid Supplementation on Early Inflammatory Responses during Cutaneous Wound Healing. Mediat. Inflamm. 2010, 2010, e342328. https://doi.org/10.1155/2010/342328
**8.** Sezik, E. et al. Traditional Medicine in Turkey X. Folk Medicine in Central Anatolia. J. Ethnopharmacol. 2001, 75, 95-115. https://doi.org/10.1016/S0378-8741(00)00399-8.
**9.** Hrynyk i Neufeld, 2014. Hrynyk M, Neufeld RJ. Insulin and wound healing. Burns. 2014, 40(8), 1433-46. doi: 10.1016/j.burns.2014.03.020
**10.** Wang, B. et al. The Preparation of Lactoferrin/Magnesium Silicate Lithium Injectable Hydrogel and Application in Promoting Wound Healing. Int. J. Biol. Macromol. 2022, 220, 1501-1511. https://doi.org/10.1016/j.ijbiomac.2022.09.126
**11.** Wang, Y.W. and Jones, P.J.H. (2004) Conjugated Linoleic Acid and Obesity Control: Efficacy and Mechanisms. International Journal of Obesity and Related Metabolic Disorders, 28, 941- 955. http://dx.doi.org/10.1038/sj.ijo.0802641
**12.** Zhang, Z.; Lv, L. Effect of Local Insulin Injection on Wound Vascularization in Patients with Diabetic Foot Ulcer. Exp. Ther. Med. 2016, 11, 397-402. https://doi.org/10.3892/etm.2015.2917
**13.** Zhang, X.; Wei, P.; Yang, Z.; Liu, Y.; Yang, K.; Cheng, Y.; Yao, H.; Zhang, Z. Current Progress and Outlook of Nano-Based Hydrogel Dressings for Wound Healing. Pharmaceutics 2023, 15, 68. https://doi.org/10.3390/pharmaceutics15010068
**14.** Zhao, L. et al. et al. pH and Glucose Dual-Responsive Injectable Hydrogels with Insulin and Fibroblasts as Bioactive Dressings for Diabetic Wound Healing. ACS Appl. Mater. Interfaces 2017, 9, 37563-37574. https://doi.org/10.1021/acsami.7b09395.

## Claims

1. A hydrogel dressing comprising at least one layer of a hydrogel matrix with at least one bioactive substance, wherein the said hydrogel matrix comprises from 4 to 8 wt.% of an aqueous solution of polyvinyl alcohol (PVA) and 4 wt.% of an aqueous solution of sodium tetraborate (borax), and wherein the bioactive substance is sheep milk in an amount of from 5 to 20 wt.% and/or camel milk in an amount of from 2.5 to 15 wt.%.

2. The hydrogel dressing according to claim 1, wherein the hydrogel matrix comprises 4 wt.% of an aqueous solution of polyvinyl alcohol (PVA).

3. The hydrogel dressing according to claim 1, wherein the sheep milk and/or camel milk is in the form of a lyophilisate.

4. The hydrogel dressing according to claim 1 or 2, wherein said dressing comprises three layers, wherein:
an inner layer closest to the wound comprises a lyophilisate of sheep milk in an amount of from 10 to 20 wt.% and a lyophilisate of camel milk in an amount of from 10 to 15 wt.%,
a middle layer comprises from 5 to 15 wt.% of a lyophilisate of sheep milk and from 2.5 to 10 wt.% of a lyophilisate of camel milk,
an outer layer comprises exclusively the hydrogel matrix.

5. The hydrogel dressing according to claim 4, wherein said dressing comprises three layers, wherein:
the inner layer closest to the wound comprises a lyophilisate of sheep milk in an amount of 15 wt.% and a lyophilisate of camel milk in an amount of 10 wt.%,
the middle layer contains a lyophilisate of sheep milk in an amount of 5 wt.% and a lyophilisate of camel milk in an amount of 5 wt.%,
the outer layer comprises exclusively the hydrogel matrix.

6. A method of producing a hydrogel dressing as defined in any one of claims 1 to 5 comprising the following steps:
a) preparing a bioactive substance in the form of a lyophilisate of sheep milk and/or a lyophilisate of camel milk at a temperature in the range from -90°C to -60°C,
b) preparing a hydrogel matrix by mixing an aqueous solution of polyvinyl alcohol (PVA) prepared at a temperature in the range from 60°C to 80°C and an aqueous solution of sodium tetraborate prepared at a temperature in the range from 35°C to 45°C, and subsequently dropwise adding the aqueous solution of sodium tetraborate to the aqueous solution of polyvinyl alcohol and mixing until a gel is obtained,
c) adding the bioactive substance to the hydrogel matrix in the form of sheep milk in an amount from 5 to 20 wt.% and/or camel milk in an amount from 2.5 to 15 wt.%.

7. The method of producing a hydrogel dressing according to claim 6, wherein the bioactive substance in step (a) is prepared at a temperature of -80°C.

8. The method of producing a hydrogel dressing according to claim 6, wherein the borax solution in step b) is prepared at a temperature of 40°C.
